(19) Europäisches Patentamt
European Patent Office
Office européen des brevets

(11) EP 4 410 268 A1

(12) EUROPEAN PATENT APPLICATION
published in accordance with Art. 153(4) EPC

(43) Date of publication:
**07.08.2024 Bulletin 2024/32**

(21) Application number: **22875949.4**

(22) Date of filing: **21.09.2022**

(51) International Patent Classification (IPC):
***A61K 8/25*** *(2006.01)* ***A61K 8/24*** *(2006.01)*
***A61K 8/31*** *(2006.01)* ***A61K 8/33*** *(2006.01)*
***A61K 8/34*** *(2006.01)* ***A61K 8/37*** *(2006.01)*
***A61K 8/41*** *(2006.01)* ***A61Q 5/10*** *(2006.01)*

(52) Cooperative Patent Classification (CPC):
**A61K 8/24; A61K 8/25; A61K 8/31; A61K 8/33; A61K 8/34; A61K 8/37; A61K 8/41; A61K 8/49; A61Q 5/10**

(86) International application number:
**PCT/JP2022/035067**

(87) International publication number:
**WO 2023/054079 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2021 JP 2021161671**

(71) Applicant: **Kao Corporation**
**Tokyo 103-8210 (JP)**

(72) Inventors:
- **ISHIMARU, Wakana**
  **Wakayama-shi, Wakayama 640-8580 (JP)**
- **SHIMIZU, Kohei**
  **Wakayama-shi, Wakayama 640-8580 (JP)**
- **NONAKA, Nobuhiro**
  **Wakayama-shi, Wakayama 640-8580 (JP)**
- **FUKUHARA, Yoshiki**
  **Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

# (54) SOLID COMPOSITION FOR DYEING KERATIN

(57) The present invention relates to a solid composition for dyeing keratin, containing a dye and a coated solid alkaline agent, in which at least a part of a surface of the solid alkaline agent is coated with a coating material having a melting point of 30°C or more and containing one or more selected from an alcohol, an ether, an ester, and a hydrocarbon.

EP 4 410 268 A1

## Description

### Field of the Invention

The present invention relates to a solid composition for dyeing keratin and a method for producing the same, and a hair dye kit.

### Background of the Invention

There has hitherto been disclosed a hair dyeing method with an oxidation dye obtained by mixing an agent containing an alkaline agent and an oxidation dye precursor, and an oxidizing agent.

For example, JP2019-151615A (PTL 1) discloses a hair dyeing method including the following steps (I) and (II). Step (I): a step of mixing a first agent containing an alkaline agent and an oxidation dye precursor, a second agent containing an oxidizing agent, and a third agent containing at least one dye (A) and having a pH at 25°C of 7.5 or more and 12 or less when diluted to be 10 times by mass with water; and step (II): a step of applying a mixed solution prepared in the step (I) on hair

The description of EP1752191A (PTL 2) describes a hair dye in which a solid alkalizing agent is contained in a particle core and is coated with fatty acids, lactones, acid anhydrides, water-soluble polymers, or the like, in which the solid alkalizing agent is to be added to a lightening/dyeing agent of keratin fibers, in particular human hair. When mixed with an oxidizing agent, the hair dye is able to inhibit a rapid decomposition of the oxidizing agent to make a handling easy, and is able to reduce potential danger by inhibiting a generation of oxygen gas.

### Summary of the Invention

The present invention relates to a solid composition for dyeing keratin, containing a dye and a coated solid alkaline agent, in which at least a part of a surface of the solid alkaline agent is coated with a coating material having a melting point of 30°C or more and containing one or more selected from an alcohol, an ether, an ester, and a hydrocarbon.

### Detailed Description of the Invention

In the method of PTL 1, kind and formulation of the oxidation dye precursor to be used are limited since the oxidation dye precursor decomposes by a contact with the alkaline agent during storage, depending on the kind of the oxidation dye precursor to be used.

In addition, an object of the solid alkalizing agent of PTL 2 is to adjust solubility of the solid alkalizing agent when mixed with the oxidizing agent, and fatty acids, lactones, acid anhydrides, water-soluble polymers, or the like coating the solid alkalizing agent may be dissolved with the moisture in the air during storage, and therefore storage stability in a state in which the oxidation dye precursor is in contact with the solid alkalizing agent has been insufficient.

The present invention relates to a solid composition and a method for producing the same, a hair dye kit including the solid composition, and use of the solid composition for a cosmetic, the solid composition being excellent in storage stability even when a dye having a low alkali stability and a solid alkaline agent are mixed and stored.

The present inventors have found that, in a solid composition for dyeing keratin, containing a solid alkaline agent and a dye, a solid composition excellent in storage stability thereof can be provided by coating at least a part of a surface of the solid alkaline agent with a specific coating material.

Specifically, the present invention relates to the following <1> to <5>.

<1> A solid composition for dyeing keratin, containing a dye and a coated solid alkaline agent, in which at least a part of a surface of the solid alkaline agent is coated with a coating material having a melting point of 30°C or more and containing one or more selected from an alcohol, an ether, an ester, and a hydrocarbon.

<2> A hair dye kit including the solid composition according to <1> and a liquid composition containing an oxidizing agent.

<3> A method for producing the solid composition according to <1>, including the following Steps 1 and 2.

Step 1: a step of mixing the coating material that has been melted and the solid alkaline agent to obtain a coated material of the solid alkaline agent

Step 2: a step of mixing the coated material obtained and a dye

<4> Use of a solid composition for a cosmetic, the solid composition containing a dye and a coated solid alkaline agent, in which at least a part of a surface of the solid alkaline agent is coated with a coating material having a

melting point of 30°C or more and containing one or more selected from an alcohol, an ether, an ester, and a hydrocarbon.
<5> A method for inhibiting a reaction between a dye and a coated solid alkaline agent, including using the coated solid alkaline agent in which at least a part of a surface of the solid alkaline agent is coated with a coating material containing one or more selected from an alcohol, an ether, an ester, and a hydrocarbon.

According to the present invention, a solid composition excellent in storage stability and a method for producing the same, a hair dye kit including the solid composition, use of the solid composition for a cosmetic, and a method for inhibiting a reaction between a dye and a solid alkaline agent can be provided.

[Solid Composition for Dyeing Keratin]

The solid composition for dyeing keratin of the present invention (hereinafter also simply referred to as a "solid composition") is a solid composition for dyeing keratin at 25°C (1013.25 hPa), containing a dye and a coated solid alkaline agent, in which at least a part of a surface of the solid alkaline agent is coated with a coating material having a melting point of 30°C or more and containing one or more selected from an alcohol, an ether, an ester, and a hydrocarbon. In the solid composition for dyeing keratin of the present invention, since the surface of the solid alkaline agent is coated with the coating material, a decomposition of the dye by an alkali due to a contact with the solid alkaline agent during storage in humidification can be prevented, and storage stability of the solid composition (hereinafter also simply referred to as storage stability) is thereby improved. Therefore, the surface of the solid alkaline agent may be coated with the coating material to such a degree that the storage stability improves.
In the solid composition for dyeing keratin of the present invention, even though the alkaline agent is coated with the coating material, dyeability hardly decreases. A reason thereof is presumed that, since an adhesiveness between the coating material that is relatively hydrophobic and the solid alkaline agent that is hydrophilic is not very high, a permeation of an oxidizing agent is hardly inhibited during a contact with the liquid oxidizing agent for a use for dyeing keratin.
The keratin is preferably keratin contained in human hair and more preferably keratin contained in human head hair.

[Solid Alkaline Agent]

The solid alkaline agent contained in the solid composition has a melting point of 30°C or more and an aqueous solution of the solid alkaline agent is basic. Examples of the solid alkaline agent include inorganic salts including salts of ammonia, hydroxides of alkali metals such as sodium hydroxide and potassium hydroxide, orthosilicates, pyrosilicates, metasilicates such as potassium metasilicate and sodium metasilicate, metadisilicates, phosphates such as tripotassium phosphate and trisodium phosphate, and carbonates such as sodium carbonate, potassium carbonate, sodium bicarbonate, potassium bicarbonate, and guanidine carbonate, and the solid alkaline agent is preferably an inorganic salt. The salt is preferably a sodium or potassium salt. Among the above-described solid alkaline agents, one or more selected from an orthosilicate, a pyrosilicate, a metasilicate, a phosphate, a metadisilicate, a carbonate, and a hydroxide are preferred, one or more selected from a metasilicate, a phosphate, and a carbonate are more preferred, and trisodium phosphate and/or sodium metasilicate is further preferred.
An average particle size of the solid alkaline agent before coating is preferably 10 μm or more, more preferably 20 μm or more, and further preferably 30 μm or more from a viewpoint of storage stability of the solid composition (hereinafter also simply referred to as storage stability), and is preferably 5 mm or less, more preferably 1 mm or less, further preferably 500 μm or less, and furthermore preferably 150 μm or less from a viewpoint of dyeability during using the solid composition. The average particle size of the solid alkaline agent before coating is preferably 10 μm or more and 5 mm or less, more preferably 20 μm or more and 1 mm or less, further preferably 30 μm or more and 500 μm or less, and furthermore preferably 30 μm or more and 150 μm or less.
The average particle size of the solid alkaline agent is measured by a method described in Examples.
A content of the solid alkaline agent in the solid composition is preferably 10% by mass or more, more preferably 15% by mass or more, and further preferably 20% by mass or more from a viewpoint of dyeability during using the solid composition, and is preferably 70% by mass or less, more preferably 65% by mass or less, and further preferably 60% by mass or less from a viewpoint of storage stability of the solid composition. The content of the solid alkaline agent in the solid composition is preferably 10% by mass or more and 70% by mass or less, more preferably 15% by mass or more and 65% by mass or less, and further preferably 20% by mass or more and 60% by mass or less.

[Coating Material]

The coating material to coat the solid alkaline agent contains one or more selected from an alcohol, an ether, an ester, and a hydrocarbon. As the coating material, a compound selected from any alcohol, ether, ester, and hydrocarbon may be used as long as it has a melting point of 30°C or more, and two or more compounds selected therefrom may be mixed and used. A total content of the above-described compounds in the coating material is preferably 50% by mass or more, more preferably 80% by mass or more, further preferably 90% by mass or more, and further preferably 99% by mass or more, and the total content may be 100% by mass.

The compounds may have a linear chain and may have a branched chain. The compounds may have a polyoxyalkylene group added thereto. The compounds may be alicyclic and may be aromatic.

The compounds have, generally, preferably 16 or more carbon atoms, more preferably 18 or more carbon atoms, further preferably 21 or more carbon atoms, further preferably 25 or more carbon atoms, further preferably 30 or more carbon atoms, and further preferably 35 or more carbon atoms from a viewpoint of storage stability of the solid composition, and preferably 200 or less carbon atoms, more preferably 100 or less carbon atoms, further preferably 80 or less carbon atoms, and further preferably 60 or less carbon atoms from a viewpoint of ease of production of the solid composition. The compounds have preferably 16 or more and 200 or less carbon atoms, more preferably 18 or more and 100 or less carbon atoms, further preferably 21 or more and 100 or less carbon atoms, further preferably 25 or more and 80 or less carbon atoms, further preferably 30 or more and 80 or less carbon atoms, and further preferably 35 or more and 60 or less carbon atoms.

When the coating material is an alcohol, an ether, or an ester, the compound preferably has a monovalent hydrocarbon group having preferably 14 or more carbon atoms, more preferably 16 or more carbon atoms, and further preferably 18 or more carbon atoms from a viewpoint of storage stability of the solid composition, and preferably has a monovalent hydrocarbon group having preferably 100 or less carbon atoms, more preferably 60 or less carbon atoms, further preferably 30 or less carbon atoms, and further preferably 22 or less carbon atoms from a viewpoint of ease of production of the solid composition. The monovalent hydrocarbon group has preferably 14 or more and 100 or less carbon atoms, more preferably 14 or more and 60 or less carbon atoms, further preferably 14 or more and 30 or less carbon atoms, further preferably 14 or more and 22 or less carbon atoms, further preferably 16 or more and 22 or less carbon atoms, and further preferably 18 or more and 22 or less carbon atoms. The monovalent hydrocarbon group may have a linear chain and may have a branched chain. The monovalent hydrocarbon group is preferably a saturated hydrocarbon group. The monovalent hydrocarbon group is a group obtained by removing one hydrogen atom from a hydrocarbon, and means, for example, a hydrocarbon group bonded to a hydroxy group in the case of the alcohol, a hydrocarbon group bonded to an oxygen atom in the case of the ether, and a group derived from a carboxylic acid and a group derived from an alcohol in the case of the ester.

Among the above-described compounds, an ester is preferred from a viewpoint of storage stability of the solid composition. A fatty acid is not considered to be preferred as the coating material, since it reacts with the alkaline agent to give soap and thereby its solubility to water increases.

The melting point of the coating material is 30°C or more, preferably 40°C or more, more preferably 45°C or more, further preferably 50°C or more, and further preferably 55°C or more from a viewpoint of storage stability of the solid composition, and is preferably 150°C or less, more preferably 120°C or less, and further preferably 90°C or less from a viewpoint of ease of a coating step of coating the solid alkaline agent. The melting point of the coating material is preferably 40°C or more and 150°C or less, more preferably 45°C or more and 120°C or less, further preferably 50°C or more and 120°C or less, and further preferably 55°C or more and 90°C or less.

The melting point of the coating material is measured by either of the first method, the second method, and the third method of the general test methods in accordance with Japanese standards of quasi-drug ingredients. Which method is to be employed is determined mainly depending on the melting point of the coating material, and the first method may be used when the melting point is as high as more than 75°C, the second method may be used when the melting point is 50°C or more and 75°C or less, and the third method may be used when the melting point is less than 50°C. As the melting point, a catalogue value may be used.

In order to set the melting point of the coating material to be 30°C or more, the melting point of the compound contained in the coating material may be set to be 30°C or more.

The alcohol used for the coating material has preferably 14 or more carbon atoms, more preferably 16 or more carbon atoms, and further preferably 18 or more carbon atoms from a viewpoint of storage stability, and preferably 200 or less carbon atoms, more preferably 100 or less carbon atoms, further preferably 80 or less carbon atoms, further preferably 60 or less carbon atoms, further preferably 30 or less carbon atoms, and further preferably 22 or less carbon atoms from a viewpoint of ease of production. The alcohol has preferably 14 or more and 200 or less carbon atoms, more preferably 16 or more and 100 or less carbon atoms, further preferably 18 or more and 80 or less carbon atoms, further preferably 18 or more and 60 or less carbon atoms, further preferably 18 or more and 30 or less carbon atoms, and further preferably 18 or more and 22 or less carbon atoms. As the alcohol, a linear or branched monohydric alcohol

is preferred, and specific examples thereof include cetyl alcohol, stearyl alcohol, and behenyl alcohol.

The ether used for the coating material has preferably 25 or more carbon atoms, more preferably 30 or more carbon atoms, and further preferably 35 or more carbon atoms from a viewpoint of storage stability, and preferably 200 or less carbon atoms, more preferably 100 or less carbon atoms, further preferably 80 or less carbon atoms, and further preferably 60 or less carbon atoms from a viewpoint of ease of production. The ether has preferably 25 or more and 200 or less carbon atoms, more preferably 30 or more and 100 or less carbon atoms, further preferably 35 or more and 80 or less carbon atoms, and further preferably 35 or more and 60 or less carbon atoms. As the ether, a dialkyl ether is preferred, and specific examples thereof include distearyl ether.

The ester used for the coating material has preferably 25 or more carbon atoms, more preferably 30 or more carbon atoms, and further preferably 35 or more carbon atoms from a viewpoint of storage stability, and preferably 200 or less carbon atoms, more preferably 100 or less carbon atoms, further preferably 80 or less carbon atoms, and further preferably 60 or less carbon atoms from a viewpoint of ease of production. The ester has preferably 25 or more and 200 or less carbon atoms, more preferably 30 or more and 100 or less carbon atoms, further preferably 35 or more and 80 or less carbon atoms, and further preferably 35 or more and 60 or less carbon atoms.

Examples of a raw material alcohol of the ester used for the coating material include monohydric alcohols; and polyhydric alcohols such as glycerin, propylene glycol, trimethylolpropane, pentaerythritol, sorbitol, and sucrose. The monohydric alcohol has preferably 1 or more and 30 or less carbon atoms, more preferably 8 or more and 30 or less carbon atoms, further preferably 12 or more and 22 or less carbon atoms, further preferably 14 or more and 22 or less carbon atoms, further preferably 16 or more and 22 or less carbon atoms, and furthermore preferably 18 or more and 22 or less carbon atoms.

Examples of a raw material carboxylic acid of the ester used for the coating material include monobasic carboxylic acids, preferably monobasic fatty acids; and polybasic carboxylic acids such as adipic acid, terephthalic acid, and trimellitic acid. The monobasic carboxylic acid has preferably 1 or more and 30 or less carbon atoms, more preferably 8 or more and 30 or less carbon atoms, further preferably 12 or more and 22 or less carbon atoms, further preferably 14 or more and 22 or less carbon atoms, further preferably 16 or more and 22 or less carbon atoms, and furthermore preferably 18 or more and 22 or less carbon atoms.

The ester preferably contains one or more selected from a fatty acid ester of a monobasic fatty acid and a monohydric alcohol, a fatty acid ester of a monobasic fatty acid and a polyhydric alcohol, and a polybasic carboxylic acid ester of a polybasic carboxylic acid and a monohydric alcohol, and more preferably contains a fatty acid ester of a monobasic fatty acid and a monohydric alcohol and/or a fatty acid ester of a monobasic fatty acid and a polyhydric alcohol. Specific examples of the ester of a monobasic fatty acid and a monohydric alcohol include cetyl palmitate, stearyl stearate, and behenyl behenate, and specific examples of the ester of a monobasic fatty acid and a polyhydric alcohol include pentaerythritol tetrastearate.

The hydrocarbon used for the coating material has, for example, preferably 21 or more carbon atoms, more preferably 25 or more carbon atoms, and further preferably 30 or more carbon atoms from a viewpoint of storage stability, and preferably 200 or less carbon atoms, more preferably 100 or less carbon atoms, further preferably 80 or less carbon atoms, and further preferably 60 or less carbon atoms from a viewpoint of ease of production. The hydrocarbon has preferably 21 or more and 200 or less carbon atoms, more preferably 25 or more and 100 or less carbon atoms, further preferably 30 or more and 80 or less carbon atoms, and further preferably 30 or more and 60 or less carbon atoms. Examples of the hydrocarbon used for the coating material include alkanes and paraffines, and specifically include Paraffine Wax 140 and Paraffine Wax 9ND.

From a viewpoint of storage stability of the solid composition, the coating material is preferably a compound having a low solubility to water. Therefore, a solubility of the coating material to 100 g of water at 25°C (1013.25 hPa) is preferably 100 mg/100 g or less, more preferably 10 mg/100 g or less, further preferably 1 mg/100 g or less, and furthermore preferably 0.1 mg/100 g or less. The lower limit of the solubility is 0 mg/100 g (insoluble).

For the measurement of the solubility, for example, Journal of The Chemical Society of Japan, 1985, No.11, p. 2116-2119 and 1982, No.11, p. 1830-1834, etc. may be referred.

A mass rate of the coating material relative to the mass of the solid alkaline agent (coating material/solid alkaline agent) is preferably 0.01 or more, more preferably 0.05 or more, further preferably 0.08 or more, and further preferably 0.15 or more from a viewpoint of storage stability of the solid composition, and is preferably 1 or less, more preferably 0.5 or less, and further preferably 0.3 or less from a viewpoint of dyeability during using the solid composition. The mass rate is preferably 0.01 or more and 1 or less, more preferably 0.05 or more and 0.5 or less, further preferably 0.08 or more and 0.3 or less, and further preferably 0.15 or more and 0.3 or less.

The content of the coating material in the solid composition is preferably 1% by mass or more, more preferably 3% by mass or more, further preferably 5% by mass or more, and further preferably 8% by mass or more from a viewpoint of storage stability of the solid composition, and is preferably 30% by mass or less, more preferably 20% by mass or less, and further preferably 15% by mass or less from a viewpoint of dyeability during using the solid composition. The content of the coating material in the solid composition is preferably 1% by mass or more and 30% by mass or less,

more preferably 3% by mass or more and 20% by mass or less, further preferably 5% by mass or more and 15% by mass or less, and further preferably 8% by mass or more and 15% by mass or less.

The average particle size of the solid alkaline agent after coating is preferably 10 μm or more, more preferably 20 μm or more, and further preferably 30 μm or more from a viewpoint of storage stability, and is preferably 5 mm or less, more preferably 1 mm or less, and further preferably 500 μm or less from a viewpoint of dyeability during using the solid composition. The average particle size of the solid alkaline agent after coating is preferably 10 μm or more and 5 mm or less, more preferably 20 μm or more and 1 mm or less, and further preferably 30 μm or more and 500 μm or less.

The average particle size of the solid alkaline agent after coating is measured by a method described in Examples.

[Dye]

The dye preferably contains one or more selected from a precursor, a coupler, and a direct dye, and more preferably contains a precursor and a coupler. Storage stability of the above-described dyes may decrease due to decomposition or the like, when the dyes contact with the solid alkaline agent.

The dye is preferably solid at 25°C (1013.25 hPa).

A content of the dye in the solid composition is preferably 0.03% by mass or more, more preferably 0.05% by mass or more, further preferably 0.1% by mass or more, and further preferably 0.3% by mass or more from a viewpoint of obtaining sufficient dyeability, and is preferably 30% by mass or less, more preferably 20% by mass or less, further preferably 10% by mass or less, and furthermore preferably 5% by mass or less from a viewpoint of storage stability of the solid composition. The content of the dye in the solid composition is preferably 0.03% by mass or more and 30% by mass or less, more preferably 0.05% by mass or more and 20% by mass or less, further preferably 0.1% by mass or more and 10% by mass or less, and further preferably 0.3% by mass or more and 5% by mass or less.

(Precursor)

The precursor is also called as an oxidation dye precursor, and is oxidized by oxygen generated from an oxidizing agent by an action of the alkaline agent and reacts with a dye coupler such as a coupler as mentioned later to give an oxidation dye.

Examples of the precursor include paraphenylenediamine compounds such as paraphenylenediamine, orthochloroparaphenylenediamine, N-phenylparaphenylenediamine, N,N-bis(hydroxyethyl)-paraphenylenediamine, 2-hydroxyethylparaphenylenediamine, 2-methoxymethylparaphenylenediamine, N-methoxyethyl-paraphenylenediamine, PEG-3,2,2'-paraphenylenediamine, 2,6-dimethyl-paraphenylenediamine; aminophenol compounds such as 3-methyl-4-aminophenol, paraaminophenol, paramethylaminophenol, orthoaminophenol, 2-aminomethyl-4-aminophenol, 2-(2-hydroxyethyl aminomethyl)-4-aminophenol, 2-amino-5-methylphenol, 2-amino-6-methylphenol, 2-amino-5-acetamidophenol; 4,4'-diaminodiphenylamine, 1,3-bis(N-(2-hydroxyethyl)-N-(4-aminophenyl)amino)-2-propanol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, toluene-2,5-diamine, 4-amino-metacresol, hydroxyethoxyaminopyrazolopyridine, and 2,3-diaminodihydroxypyrazolopyrazolone; and salts thereof.

Among these, the precursor contained in the solid composition of the present invention is preferably one or more selected from a phenylenediamine compound, an aminophenol compound, and a salt thereof, more preferably 2-methoxymethylparaphenylenediamine, N-methoxyethyl-paraphenylenediamine, paraaminophenol, and a salt thereof, and further preferably 2-methoxymethylparaphenylenediamine and a salt thereof. Examples of the salt include acetate, hydrochloride, and sulfate.

The precursor may be used alone or in combination of two or more. A content of the precursor in the solid composition is preferably 0.03% by mass or more, more preferably 0.05% by mass or more, further preferably 0.1% by mass or more, and further preferably 0.3% by mass or more from a viewpoint of obtaining sufficient dyeability, and is preferably 30% by mass or less, more preferably 20% by mass or less, further preferably 10% by mass or less, and further preferably 5% by mass or less from a viewpoint of storage stability of the solid composition. The content of the precursor in the solid composition is preferably 0.03% by mass or more and 30% by mass or less, more preferably 0.05% by mass or more and 20% by mass or less, further preferably 0.1% by mass or more and 10% by mass or less, and further preferably 0.3% by mass or more and 5% by mass or less.

(Coupler)

Examples of the coupler include resorcinol, 2-methylresorcinol, 1-naphthol, 1,5-dihydroxynaphthalene, 5-aminoorthocresol, metaphenylenediamine, metaaminophenol, 2,4-diaminophenoxyethanol, 2-methyl-5-amino-6-chlorophenol, 2-amino-4-hydroxyethylaminoanisole, 5-amino-6-chloro-o-cresol, 3-amino-2,4-dichlorophenol, 2,6-diaminopyridine, 2-methyl-5-hydroxyethylaminophenol, 2-amino-3-hydroxypyridine, paraaminophenol, orthoaminophenol, 2-amino-4-(2-hydroxyethylamino)anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, 2-methyl-5-aminophenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, 4-chlororesorcinol, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, and salts thereof.

Among these, the coupler preferably contains one or more selected from an aromatic amine compound having an aromatic amine as the skeleton thereof, the aromatic amine having one or more amino groups in an aromatic ring; a phenol compound having a phenol skeleton having one or more hydroxy groups in an aromatic ring; a pyridine compound having a pyridine skeleton; and a salt thereof. The coupler contained in the solid composition of the present invention is preferably one or more selected from 1,3-bis(2,4-diaminophenoxy)propane, 2-chloro-3-amino-6-methylphenol, 4-chlororesorcinol, 2-methylresorcinol, 2-amino-3-hydroxypyridine, 2,4-diaminophenoxyethanol, 2-methyl-5-amino-6-chlorophenol, 2-amino-4-hydroxyethylaminoanisole, 5-amino-6-chloro-o-cresol, 3-amino-2,4-dichlorophenol, and a salt thereof, and more preferably 1,3-bis(2,4-diaminophenoxy)propane, 2-chloro-3-amino-6-methylphenol, or a salt thereof. Examples of the salt include acetate, hydrochloride, and sulfate.

The coupler may be used alone or in combination of two or more. A content of the coupler in the solid composition is preferably 0.03% by mass or more, more preferably 0.05% by mass or more, further preferably 0.1% by mass or more, and further preferably 0.3% by mass or more from a viewpoint of obtaining sufficient dyeability, and is preferably 30% by mass or less, more preferably 20% by mass or less, further preferably 10% by mass or less, and further preferably 5% by mass or less from a viewpoint of storage stability of the solid composition. The content of the coupler in the solid composition is preferably 0.03% by mass or more and 30% by mass or less, more preferably 0.05% by mass or more and 20% by mass or less, further preferably 0.1% by mass or more and 10% by mass or less, and further preferably 0.3% by mass or more and 5% by mass or less.

(Direct Dye)

Examples of the direct dye include azo dyes, anionic dyes, cationic dyes, and neutral dyes. Examples of the azo dye include a dye represented by any of the following structural formulae (A-1), (A-2), and (A-3).

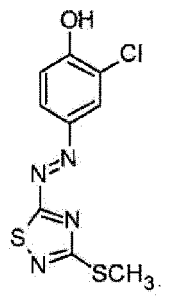
(A-1)

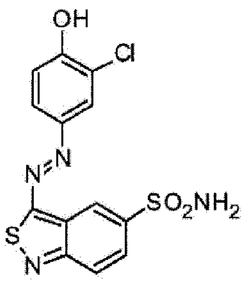
(A-2)

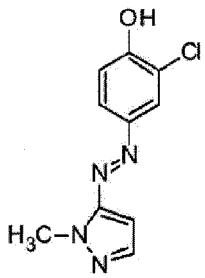
(A-3)

Examples of the anionic dye include Acid Black 1, Acid Blue 1, Acid Blue 3, Food Blue No. 2, Food Blue No. 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, Acid Orange 24, Acid Green 25, Solvent Green 7, Solvent Red 73, Acid Red 95, Solvent Red 43, Solvent Red 48, Acid Red 33, Solvent Violet 13, Acid Yellow 73, Food Red No. 17, Food Red No. 1, Food Yellow No. 3, Food Blue No. 2, Food Black No. 1, Food Black No. 2, Disperse Black 9, Disperse Violet 1, and alkali metal salts thereof (sodium salt or potassium salt).

Examples of the cationic dye include Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12, Basic Red 22, Basic Red 76, Basic Violet 1, Basic Violet 2, Basic Violet 3, Basic Violet 10, Basic Violet 14, Basic Yellow 57, Basic Red 51, Basic Yellow 87, Basic Blue 17, and Basic Orange 31.

Examples of the neutral dye including a nitro dye include HC Blue 2, HC Blue 4, HC Blue 5, HC Blue 6, HC Blue 7, HC Blue 8, HC Blue 9, HC Blue 10, HC Blue 11, HC Blue 12, HC Blue 13, HC Brown 1, HC Brown 2, HC Green 1, HC Orange 1, HC Orange 2, HC Orange 3, HC Orange 5, HC Red BN, HC Red 1, HC Red 3, HC Red 7, HC Red 8, HC Red 9, HC Red 10, HC Red 11, HC Red 13, HC Red 54, HC Red 14, HC Violet BS, HC Violet 1, HC Violet 2, HC Yellow 2, HC Yellow 4, HC Yellow 5, HC Yellow 6, HC Yellow 7, HC Yellow 8, HC Yellow 9, HC Yellow 10, HC Yellow 11, HC Yellow 12, HC Yellow 13, HC Yellow 14, HC Yellow 15, 2-amino-6-chloro-4-nitrophenol, picramic acid, 1,2-diamino-4-nitrobenzene, 1,4-diamino-2-nitrobenzene, 3-nitro-4-aminophenol, 1-hydroxy-2-amino-3-nitrobenzene, 2-hydroxyethyl picramic acid, 3-nitro-p-hydroxyethylaminophenol, 4-hydroxypropylamino-3-nitrophenol, and N,N-bis(2-hydroxyethyl)-2'-nitro-p-phenylenediamine.

The direct dye may be used alone or in combination of two or more. A content of the direct dye in the solid composition is preferably 0.03% by mass or more, more preferably 0.05% by mass or more, further preferably 0.1% by mass or more, and further preferably 0.3% by mass or more from a viewpoint of obtaining sufficient dyeability, and is preferably 30% by mass or less, more preferably 20% by mass or less, further preferably 10% by mass or less, and further preferably 5% by mass or less from a viewpoint of storage stability of the solid composition.

A method for coating the solid alkaline agent by the coating material will be described in detail in [Production Method of Solid Composition] below.

[Other Components]

The solid composition may contain a surface modifier, a balancing agent, a fluidity enhancer, a chelating reagent, an oil, or the like in addition to the dye and the coated solid alkaline agent, in which at least a part of a surface of the solid alkaline agent is coated with the coating material as described above.

By coating the surface of the solid alkaline agent, which has been coated with the coating material, with the surface modifier, storage stability can be further improved. As the surface modifier, organic powder and inorganic powder are preferred. Examples of the organic powder include monosaccharides or polysaccharides such as glucose, fructose, lactose, maltose, sucrose, dextrin, maltodextrin, cyclodextrin, maltose, fructose, trehalose, cellulose, cornstarch, tapioca starch, rice starch, wheat starch, potato starch, erythritol, and mannitol. Examples of the inorganic powder include silica, zeolite, bentonite, kaolin, calcium silicate, sodium sulfate, magnesium sulfate, magnesium oxide, zinc oxide, calcium stearate, magnesium stearate, magnesium oxide, sodium polyphosphate, and sodium phosphate.

An average particle size of the surface modifier is preferably 100 μm or less, more preferably 50 μm or less, and further preferably 30 μm or less from a viewpoint of further coating the surface of the solid alkaline agent having been coated with the coating material.

A mass rate of the surface modifier relative to the mass of the solid alkaline agent (surface modifier/solid alkaline agent) is preferably 0.05 or more, more preferably 0.1 or more, and further preferably 0.15 or more from a viewpoint of storage stability of the solid composition, and is preferably 1 or less, more preferably 0.5 or less, and further preferably 0.3 or less from a viewpoint of dyeability during using the solid composition. The mass rate is preferably 0.05 or more and 1 or less, more preferably 0.1 or more and 0.5 or less, and further preferably 0.15 or more and 0.3 or less.

Examples of the balancing agent include cornstarch. Examples of the fluidity enhancer include silica. Examples of the chelating reagent include ethylenediaminetetraacetic acid (EDTA). Examples of the oil include liquid oil having a melting point of less than 30°C and isopropyl myristate. When the solid composition of the present invention contains the balancing agent, a content of the balancing agent is preferably 5% by mass or more and more preferably 8% by mass or more, and preferably 75% by mass or less and more preferably 60% by mass or less, from a viewpoint of storage stability of the solid composition. The content of the balancing agent is preferably 5% by mass or more and 75% by mass or less, and more preferably 8% by mass or more and 60% by mass or less.

A content of water in the solid composition of the present invention is, from a viewpoint of storage stability, preferably 10% by mass or less, more preferably 3% by mass or less, further preferably 1% by mass or less, and furthermore preferably 0.5% by mass or less, and the lower limit thereof is 0% by mass or more and preferably 0.001% by mass or more. The content of water in the solid composition is preferably 0% by mass or more and 10% by mass or less, more preferably 0.001% by mass or more and 3% by mass or less, further preferably 0.001% by mass or more and 1% by mass or less, and furthermore preferably 0.001% by mass or more and 0.5% by mass or less.

Examples of the solid composition include powder and briquette (tablet), and a powder composition is preferred.

An average particle size of the powder is the same as the preferable range of the average particle size of the alkaline agent after coating.

In the case of a compression-molded briquette (tablet), a maximum length is preferably 1 mm or more, more preferably 3 mm or more, and further preferably 5 mm or more, and preferably 50 mm or less, more preferably 30 mm or less, and further preferably 10 mm or less. The maximum length means a number average value of randomly sampled 50 long diameters (length of a straight line, connecting points that are the most distant from each other on a surface of a particle) observed with an optical microscope.

The solid composition of the present invention is preferably a hair dye composition and more preferably a solid composition for a hair dye.

[Jar-Type Container]

The solid composition of the present invention is preferably used while contained in a jar-type container.
The jar-type container is preferably a jar-type container that stores a content, and includes a bottomed-cylindrical or prismatic container main body having an opening part at an upper end part thereof, and a lid body screwed to the container main body (fit together in a screw-like manner).
Such a container is utilized by removing the lid body, and directly scooping the content while putting a finger into the container main body or scooping the content with an attached spatula.
The lid body and the main body may be fit together or openably/closably connected with a hinge. In the jar-type container, the content is easy to contact with the air during the use as described above, but it is not a problem since the solid composition of the present invention has an excellent storage stability even when it contacts with the moisture in the air.

[Hair Dye Kit]

The solid composition of the present invention can be applied as a hair dye kit including the solid composition and a liquid composition containing an oxidizing agent which are separately packed.
A hair dyeing mechanism using the hair dye kit is considered to be as below.
First, by an external force applied during mixing the solid composition and the liquid composition, the solid alkaline agent and the coating material are separated, and the solid alkaline agent dissolves into water contained in the liquid composition to give alkaline liquid. The alkaline liquid opens the cuticle of hair and thereby mixed solution of the solid composition and the liquid composition permeates into the hair. The alkaline liquid reacts with the oxidizing agent to produce oxygen, and bleaches melanin pigments existing in the hair and oxidizes a precursor at the same time. The oxidized precursor reacts with a dye coupler such as a coupler and thereby produces an oxidation dye to develop color. Molecules of the colored oxidation dye increase in size by polymerization and thereby do not get out of gaps of the cuticle, the dye fixes inside the hair, and thus hair dyeing is completed. At the same time, a direct dye also permeates into the hair and develops color.
The solid composition of the present invention is preferably used for hair dyeing by being mixed with a liquid composition containing an oxidizing agent before applying.
A content percentage of the solid composition of the present invention in the total amount of the solid composition of the present invention and the liquid composition is preferably 1% by mass or more and more preferably 5% by mass or more, and preferably 20% by mass or less and more preferably 15% by mass or less. The content percentage of the solid composition is preferably 1% by mass or more and 20% by mass or less, and more preferably 5% by mass or more and 15% by mass or less.
Examples of the oxidizing agent contained in the liquid composition include peroxides such as hydrogen peroxide water, urea peroxide, and melamine peroxide, and the oxidizing agent is preferably hydrogen peroxide water. When the hydrogen peroxide water is used, a concentration of hydrogen peroxide in the liquid composition is preferably 1% by mass or more and more preferably 2% by mass or more from a viewpoint of reactivity with the precursor, and preferably 30% by mass or less and more preferably 20% by mass or less from a viewpoint of handleability. The concentration of hydrogen peroxide in the liquid composition is preferably 1% by mass or more and 30% by mass or less, and more preferably 2% by mass or more and 20% by mass or less.
Hair dyeing is performed at the temperature of preferably equal to or more than room temperature and more preferably 30°C or more, and preferably 45°C or less. A hair dyeing period is preferably 15 minutes or more and more preferably 25 minutes or more, and preferably 45 minutes or less and more preferably 35 minutes or less. The hair dyeing period is preferably 15 minutes or more and 45 minutes or less, and more preferably 25 minutes or more and 35 minutes or less.

[Production Method of Solid Composition]

The solid composition of the present invention is preferably produced by a production method including the following Steps 1 and 2.

Step 1: a step of mixing the coating material that has been melted and the solid alkaline agent to obtain a coated material of the solid alkaline agent
Step 2: a step of mixing the coated material obtained and a dye

By heating the coating material to a temperature equal to or more than the melting point of the coating material, the coating material can be melted. A mass rate between the coating material and the solid alkaline agent, that is, the mass rate of the coating material relative to the mass of the solid alkaline agent (coating material/solid alkaline agent) is preferably 0.01 or more, more preferably 0.05 or more, further preferably 0.08 or more, and further preferably 0.15 or more from a viewpoint of storage stability of the solid composition, and is preferably 1 or less, more preferably 0.5 or less, and further preferably 0.3 or less from a viewpoint of dyeability during using the solid composition. The mass rate is preferably 0.01 or more and 1 or less, more preferably 0.05 or more and 0.5 or less, further preferably 0.08 or more and 0.3 or less, and further preferably 0.15 or more and 0.3 or less.

[Use for Cosmetics]

The solid composition for dyeing keratin, containing a dye and a coated solid alkaline agent, in which at least a part of a surface of the solid alkaline agent is coated with a coating material having a melting point of 30°C or more and containing one or more selected from an alcohol, an ether, an ester, and a hydrocarbon is preferably used for cosmetics, especially powder cosmetics. Preferable embodiments of the coating material and the solid alkaline agent are the same as those described above.

The solid composition may further contain pigments, antibacterial agents, preservatives, UV absorbers, fragrances, moisturizing ingredients, and the like.

Examples of the powder cosmetic include makeup cosmetics, and hair treatment agents such as hair dyes, permanent wave agents, and treatments, and among these, hair dyes are preferred.

[Method for Inhibiting Reaction]

By using the coated solid alkaline agent in which at least a part of a surface of the solid alkaline agent is coated with a coating material containing one or more selected from an alcohol, an ether, an ester, and a hydrocarbon, a decrease in dyeability due to a decomposition of the dye can be inhibited even when the solid alkaline agent contacts with the dye. Namely, a reaction between the dye and the solid alkaline agent can be inhibited. Preferable embodiments of the coating material and the solid alkaline agent are the same as those described above.

The present invention includes embodiments of the following <1> to <77>.

<1> A solid composition for dyeing keratin, containing a dye and a coated solid alkaline agent, in which at least a part of a surface of the solid alkaline agent is coated with a coating material having a melting point of 30°C or more and containing one or more selected from an alcohol, an ether, an ester, and a hydrocarbon.

<2> The solid composition according to <1>, in which the coating material has a melting point of 40°C or more.

<3> The solid composition according to <1> or <2>, in which the coating material has a melting point of 50°C or more.

<4> The solid composition according to any one of <1> to <3>, in which the coating material has a melting point of 55°C or more.

<5> The solid composition according to any one of <1> to <4>, in which the coating material has a melting point of 150°C or less.

<6> The solid composition according to any one of <1> to <5>, in which the coating material has a melting point of 120°C or less.

<7> The solid composition according to any one of <1> to <6>, in which the alcohol contains a linear or branched monohydric alcohol.

<8> The solid composition according to any one of <1> to <7>, in which the ether contains a dialkyl ether.

<9> The solid composition according to any one of <1> to <8>, in which the ester contains one or more selected from a fatty acid ester of a monobasic fatty acid and a monohydric alcohol, a fatty acid ester of a monobasic fatty acid and a polyhydric alcohol, and a polybasic carboxylic acid ester of a polybasic carboxylic acid and a monohydric alcohol.

<10> The solid composition according to any one of <1> to <9>, in which the ester contains a fatty acid ester of a monobasic fatty acid and a monohydric alcohol and/or a fatty acid ester of a monobasic fatty acid and a polyhydric alcohol.

<11> The solid composition according to any one of <1> to <10>, in which the hydrocarbon contains an alkane or a paraffine.

<12> The solid composition according to any one of <1> to <11>, in which a mass rate of the coating material relative to a mass of the solid alkaline agent (coating material/solid alkaline agent) is 0.01 or more and 1 or less.

<13> The solid composition according to any one of <1> to <12>, in which the mass rate of the coating material relative to the mass of the solid alkaline agent (coating material/solid alkaline agent) is 0.05 or more and 0.5 or less.

<14> The solid composition according to any one of <1> to <13>, in which the mass rate of the coating material

relative to the mass of the solid alkaline agent (coating material/solid alkaline agent) is 0.08 or more and 0.3 or less.

<15> The solid composition according to any one of <1> to <14>, in which the mass rate of the coating material relative to the mass of the solid alkaline agent (coating material/solid alkaline agent) is 0.15 or more and 0.3 or less.

<16> The solid composition according to any one of <1> to <15>, in which the solid alkaline agent contains one or more selected from an orthosilicate, a pyrosilicate, a metasilicate, a phosphate, a metadisilicate, a carbonate, and a hydroxide.

<17> The solid composition according to any one of <1> to <16>, in which the solid alkaline agent contains one or more selected from a metasilicate, a phosphate, and a carbonate.

<18> The solid composition according to any one of <1> to <17>, in which the solid alkaline agent contains one or more selected from trisodium phosphate and sodium metasilicate.

<19> The solid composition according to any one of <1> to <18>, in which a solubility of the coating material to water at 25°C is 100 mg/100 g or less.

<20> The solid composition according to any one of <1> to <19>, in which the solubility of the coating material to water at 25°C is 10 mg/100 g or less.

<21> The solid composition according to any one of <1> to <20>, in which the solubility of the coating material to water at 25°C is 1 mg/100 g or less.

<22> The solid composition according to any one of <1> to <21>, in which the solubility of the coating material to water at 25°C is 0.1 mg/100 g or less.

<23> The solid composition according to any one of <1> to <22>, in which a compound used for the coating material has 16 or more carbon atoms.

<24> The solid composition according to any one of <1> to <23>, in which the compound used for the coating material has 18 or more carbon atoms.

<25> The solid composition according to any one of <1> to <24>, in which the compound used for the coating material has 21 or more carbon atoms.

<26> The solid composition according to any one of <1> to <25>, in which the compound used for the coating material has 25 or more carbon atoms.

<27> The solid composition according to any one of <1> to <26>, in which the compound used for the coating material has 30 or more carbon atoms.

<28> The solid composition according to any one of <1> to <27>, in which the compound used for the coating material has 200 or less carbon atoms.

<29> The solid composition according to any one of <1> to <28>, in which the compound used for the coating material has 100 or less carbon atoms.

<30> The solid composition according to any one of <1> to <29>, in which the compound used for the coating material has 80 or less carbon atoms.

<31> The solid composition according to any one of <1> to <22>, in which a compound used for the coating material has a monovalent hydrocarbon group having 14 or more carbon atoms.

<32> The solid composition according to any one of <1> to <22>, in which a compound used for the coating material has a monovalent hydrocarbon group having 16 or more carbon atoms.

<33> The solid composition according to any one of <1> to <22>, in which a compound used for the coating material has a monovalent hydrocarbon group having 18 or more carbon atoms.

<34> The solid composition according to any one of <1> to <22>, in which a compound used for the coating material has a monovalent hydrocarbon group having 60 or less carbon atoms.

<35> The solid composition according to any one of <1> to <22>, in which a compound used for the coating material has a monovalent hydrocarbon group having 30 or less carbon atoms.

<36> The solid composition according to any one of <1> to <22>, in which a compound used for the coating material has a monovalent hydrocarbon group having 22 or less carbon atoms.

<37> The solid composition according to any one of <1> to <36>, further containing a surface modifier.

<38> The solid composition according to <37>, in which a mass rate of the surface modifier relative to the mass of the solid alkaline agent (surface modifier/solid alkaline agent) is 0.05 or more and 1 or less.

<39> The solid composition according to <37> or <38>, in which the mass rate of the surface modifier relative to the mass of the solid alkaline agent (surface modifier/solid alkaline agent) is 0.1 or more and 0.5 or less.

<40> The solid composition according to any one of <37> to <39>, in which the surface modifier is organic powder or inorganic powder.

<41> The solid composition according to <40>, in which the organic powder is cornstarch.

<42> The solid composition according to <40>, in which the inorganic powder is silica.

<43> The solid composition according to any one of <1> to <42>, in which the dye contains one or more selected from a precursor, a coupler, and a direct dye.

<44> The solid composition according to <43>, in which the dye contains a precursor and a coupler.

<45> The solid composition according to <43> or <44>, in which the precursor contains one or more selected from a phenylenediamine compound, an aminophenol compound, and a salt thereof.

<46> The solid composition according to any one of <43> to <45>, in which the precursor contains one or more selected from 2-methoxymethylparaphenylenediamine, N-methoxyethyl-paraphenylenediamine, paraaminophenol, and a salt thereof.

<47> The solid composition according to any one of <43> to <46>, in which the coupler contains one or more selected from an aromatic amine compound, a phenol compound, a pyridine compound, and a salt thereof.

<48> The solid composition according to any one of <43> to <47>, in which the coupler contains one or more selected from 1,3-bis(2,4-diaminophenoxy)propane, 2-chloro-3-amino-6-methylphenol, 4-chlororesorcinol, 2-methylresorcinol, 2-amino-3-hydroxypyridine, 2,4-diaminophenoxyethanol, 2-methyl-5-amino-6-chlorophenol, 2-amino-4-hydroxyethylaminoanisole, 5-amino-6-chloro-o-cresol, 3-amino-2,4-dichlorophenol, and a salt thereof.

<49> The solid composition according to any one of <1> to <48>, in which an average particle size of the solid alkaline agent before coating is 10 μm or more and 5 mm or less.

<50> The solid composition according to any one of <1> to <49>, in which the average particle size of the solid alkaline agent before coating is 20 μm or more and 1 mm or less.

<51> The solid composition according to any one of <1> to <50>, in which the average particle size of the solid alkaline agent before coating is 30 μm or more and 500 μm or less.

<52> The solid composition according to any one of <1> to <51>, in which the average particle size of the solid alkaline agent before coating is 30 μm or more and 150 μm or less.

<53> The solid composition according to any one of <1> to <48>, in which an average particle size of the solid alkaline agent after coating is 10 μm or more and 5 mm or less.

<54> The solid composition according to any one of <1> to <48>, in which an average particle size of the solid alkaline agent after coating is 20 μm or more and 1 mm or less.

<55> The solid composition according to any one of <1> to <48>, in which an average particle size of the solid alkaline agent after coating is 30 μm or more and 500 μm or less.

<56> The solid composition according to any one of <1> to <55>, in which a content of the coating material in the solid composition is 1% by mass or more and 30% by mass or less.

<57> The solid composition according to any one of <1> to <56>, in which the content of the coating material in the solid composition is 3% by mass or more and 20% by mass or less.

<58> The solid composition according to any one of <1> to <57>, in which the content of the coating material in the solid composition is 5% by mass or more and 15% by mass or less.

<59> The solid composition according to any one of <1> to <58>, in which a content of the solid alkaline agent in the solid composition is 10% by mass or more and 70% by mass or less.

<60> The solid composition according to any one of <1> to <59>, in which the content of the solid alkaline agent in the solid composition is 15% by mass or more and 65% by mass or less.

<61> The solid composition according to any one of <1> to <60>, in which the content of the solid alkaline agent in the solid composition is 20% by mass or more and 60% by mass or less.

<62> The solid composition according to any one of <1> to <61>, in which a content of the dye in the solid composition is 0.03% by mass or more and 30% by mass or less.

<63> The solid composition according to any one of <1> to <62>, in which the content of the dye in the solid composition is 0.05% by mass or more and 20% by mass or less.

<51> The solid composition according to any one of <1> to <63>, in which the content of the dye in the solid composition is 0.1% by mass or more and 10% by mass or less.

<64> The solid composition according to any one of <1> to <51>, in which a content of the dye in the solid composition is 0.3% by mass or more and 5% by mass or less.

<65> The solid composition according to any one of <1> to <64>, in which the content of water in the solid composition is 10% by mass or less.

<66> The solid composition according to any one of <1> to <65>, in which the content of water in the solid composition is 1% by mass or less.

<67> The solid composition according to any one of <1> to <66>, in which the content of water in the solid composition is 0.5% by mass or less.

<68> The solid composition according to any one of <1> to <67>, which is a powder composition.

<69> The solid composition according to any one of <1> to <68>, for a hair dye.

<70> The solid composition according to any one of <1> to <69>, stored in a jar-type container.

<71> A hair dye kit including the solid composition according to any one of <1> to <70> and a liquid composition containing an oxidizing agent.

<72> The hair dye kit according to <71>, in which the oxidizing agent contains hydrogen peroxide water.

<73> A method for producing the solid composition according to any one of <1> to <69>, including the following

Steps 1 and 2.

Step 1: a step of mixing the coating material that has been melted and the solid alkaline agent to obtain a coated material of the solid alkaline agent
Step 2: a step of mixing the coated material obtained and a dye

<74> Use of a solid composition for a cosmetic, the solid composition containing a dye and a coated solid alkaline agent, in which at least a part of a surface of the solid alkaline agent is coated with a coating material having a melting point of 30°C or more and containing one or more selected from an alcohol, an ether, an ester, and a hydrocarbon.
<75> The use according to <74>, in which the cosmetic is a hair treatment agent.
<76> The use according to <74> or <75>, in which the cosmetic is a hair dye.
<77> A method for inhibiting a reaction between a dye and a coated solid alkaline agent, including using the coated solid alkaline agent in which at least a part of a surface of the solid alkaline agent is coated with a coating material containing one or more selected from an alcohol, an ether, an ester, and a hydrocarbon.

Examples

In the following Examples and Comparative Examples, "%" is "% by mass" unless otherwise indicated. The measurements of property values were performed by the following methods.

[Measurement Methods]

<Average Particle Sizes of Solid Alkaline Agent and Coated Solid Alkaline Agent>

Using standard sieves (opening: 45 to 2000 μm) specified in JIS K 8801-1:2016, 100 g of the solid alkaline agent was vibrated for 5 minutes, and then the average particle size of the solid alkaline agent was calculated from a mass distribution according to the sieve openings.
More specifically, vibration was performed for 5 minutes using sieves of 2000 μm, 1400 μm, 1000 μm, 710 μm, 500 μm, 355 μm, 250 μm, 180 μm, 125 μm, 90 μm, 63 μm, and 45 μm specified in JIS Z 8801-1:2006 (established on May 20, 2000 and last amended on November 20, 2006), then 50% average diameter was calculated for a mass distribution under the sieves by the sieving method, and the 50% average diameter was used as the average particle size. Namely, the sieves were stacked on a saucer in order starting from the sieve having the smallest opening, 100 g of the solid alkaline agent was added from above the uppermost sieve of 2000 μm, the sieves were put on a lid and attached to a low-tap sieve shaker (manufactured by Heiko Seisakusho, Ltd., tapping: 156 times/minute, rolling: 290 times/minute), followed by vibrating for 5 minutes, and then masses of the solid alkaline agents remained on each of the sieves and the saucer were measured to calculate percentages (% by mass) of the solid alkaline agent on each of the sieves. The percentages of the solid alkaline agent were added up from one on the saucer in order starting from the sieve having the smallest opening, and the particle size at which the total reached 50% by mass was used as the average particle size.
The average particle size of the coated solid alkaline agent was calculated in the same manner.
In Production Examples, the following raw materials were used.

(Solid Alkaline Agent)

·Trisodium phosphate: manufactured by Taihei Chemical Industrial Co., Ltd., average particle size 70.7 μm
·Trisodium phosphate: manufactured by Yoneyama Kagaku Kogyo Kaisha, Ltd., average particle size 170.3 μm
·Sodium metasilicate: manufactured by Silmaco NV, average particle size 86.0 μm

(Coating Material)

·Stearyl stearate: manufactured by Kao Corporation, trade name EXCEPARL SS, melting point 60°C
·Behenyl behenate: manufactured by NOF Corporation, trade name UNISTER M-2222SL, melting point 70°C
·Stearyl alcohol: manufactured by Kao Corporation, trade name KALCOL 8098, melting point 59°C

·Behenyl alcohol: manufactured by Kao Corporation, trade name KALCOL 220-98, melting point 69°C
·Paraffine Wax-140 (trade name): manufactured by Nippon Seiro Co., Ltd., hydrocarbon wax, $C_nH_{2n+2}$ (n=20 to 39), melting point 61°C
·Paraffine Wax 9ND (trade name): manufactured by Nippon Seiro Co., Ltd., hydrocarbon wax, $C_nH_{2n+2}$ (n=28 to 52), melting point 75°C
·Pentaerythritol tetrastearate: manufactured by NOF Corporation, trade name WE-476-H, melting point 62°C
·Lauryl alcohol: manufactured by Kao Corporation, trade name KALCOL 2098, melting point 24°C
·Lauric acid: manufactured by Kao Corporation, trade name LUNAC L-98, melting point 43°C
·Stearic acid: manufactured by Kao Corporation, trade name LUNAC S-98, melting point 69°C

(Surface Modifier)

·Cornstarch: manufactured by Roquette Freres, trade name MAIZE STARCH B

(Precursor)

·Precursor A160S: manufactured by Dragon Chemical (2-methoxymethylparaphenylenediamine sulfate)

(Coupler)

·Coupler A79: manufactured by Extrachem GmbH (1,3-bis(2,4-diaminophenoxy)propane tetrahydrochloride)
·Coupler A94: manufactured by Grafox Chemie Vertriebs GmbH (2-chloro-3-amino-6-methylphenol)

(Other Components)

·Cornstarch: manufactured by Roquette Freres, trade name MAIZE STARCH B
·Silica: manufactured by EVONIK Industries, trade name SIPERNAT-22
·EDTA: manufactured by FUJIFILM Wako Pure Chemical Corporation, reagent
·Isopropyl myristate: manufactured by FUJIFILM Wako Pure Chemical Corporation, reagent

[Production of Solid Composition]

(Production Example 1)

To a 2L high-speed mixer (LFS-2 manufactured by Earthtechnica Co., Ltd., rotation number of the agitator 600 rpm, rotation number of the chopper 1500 rpm, jacket hot water temperature 80°C) was added 300 g of trisodium phosphate (particle size: 70 $\mu$m) as the solid alkaline agent, powder temperature was confirmed to be 60°C or more, and 33.3 g of stearyl stearate that had been melted as the coating material was added to the mixer, followed by mixing for 3 minutes to take mixture out. The mixture obtained was received on a tray and cooled at 25°C to obtain granules of the solid alkaline agent having the surface coated with the coating material.
The granules obtained, and the precursor, the coupler, and other components indicated in Table 1 were mixed so that those had the contents indicated in Table 1, and the mixture was measures and taken into a screw tube and used as a solid composition.

(Production Examples 2 to 3)

Solid compositions were produced in the same manner as in Production Example 1 except that the content of stearyl stearate was changed as described in Table 1.

(Production Examples 4 to 9)

Solid compositions were produced in the same manner as in Production Example 1 except that the coating material was changed to the coating materials described in Table 1.

(Production Example 10)

A solid composition was produced in the same manner as in Production Example 1 except that the coating material was not used and the content of the balancing agent was changed as described in Table 1.

(Production Example 11)

A solid composition was produced in the same manner as in Production Example 1 except that stearic acid that is a fatty acid was used as the coating material.

(Production Example 12)

A solid composition was produced in the same manner as in Production Example 5 except that trisodium phosphate having an average particle size of 170.3 μm was used as the trisodium phosphate, and the contents of the components were changed as described in Table 1.

(Production Example 13)

A solid composition was produced in the same manner as in Production Example 12 except that the coating material was not used and the content of the balancing agent was changed as described in Table 1.

(Production Example 14)

A solid composition was produced in the same manner as in Production Example 7 except that the blending amount of the precursor was changed to 0.5 parts by mass, 0.8 parts by mass of A79 was used as the coupler, and the content of the balancing agent was changed as described in Table 1.

(Production Example 15)

A solid composition was produced in the same manner as in Production Example 14 except that the coating material was not used and the content of the balancing agent was changed as described in Table 1.

(Production Example 16)

A solid composition was produced in the same manner as in Production Example 5 except that sodium metasilicate was used as the solid alkaline agent, and the contents of the components were changed as described in Table 1.

(Production Example 17)

To a 2L high-speed mixer (LFS-2 manufactured by Earthtechnica Co., Ltd., rotation number of the agitator 600 rpm, rotation number of the chopper 1500 rpm, jacket hot water temperature 80°C) was added 300 g of trisodium phosphate (particle size: 170.3 μm) as the solid alkaline agent, powder temperature was confirmed to be 60°C or more, 33.3 g of stearyl alcohol that had been melted as the coating material was added to the mixer, followed by mixing for 3 minutes, and 66.7 g of cornstarch as the surface modifier was further added to the mixer, followed by mixing for 3 minutes to take mixture out. The mixture obtained was received on a tray and cooled at 25°C to obtain granules of the solid alkaline agent having the surface coated with the coating material and the surface modifier.

The granules obtained, and the precursor, the coupler, and other components indicated in Table 1 were mixed so that those had the contents indicated in Table 1, and the mixture was measured and taken into a screw tube and used as a solid composition.

Table 1-1

| | Production Examples | | 1 | 2 | 3 | 4 | 5 |
|---|---|---|---|---|---|---|---|
| PC | Solid alkaline agent | Trisodium phosphate (70.7 μm) | 55.0 | 55.0 | 55.0 | 55.0 | 55.0 |
| | | Trisodium phosphate (170.3 μm) | 0.0 | 0.0 | 0.0 | 0.0 | 0.0 |
| | | Sodium metasilicate (86.0 μm) | 0.0 | 0.0 | 0.0 | 0.0 | 0.0 |
| | Coating material | Stearyl stearate | 6.1 | 9.7 | 13.8 | 0.0 | 0.0 |
| | | Behenyl behenate | 0.0 | 0.0 | 0.0 | 6.1 | 0.0 |
| | | Stearyl alcohol | 0.0 | 0.0 | 0.0 | 0.0 | 6.1 |
| | | Behenyl alcohol | 0.0 | 0.0 | 0.0 | 0.0 | 0.0 |
| | | Paraffine Wax-140 | 0.0 | 0.0 | 0.0 | 0.0 | 0.0 |
| | | Paraffine Wax 9ND | 0.0 | 0.0 | 0.0 | 0.0 | 0.0 |
| | | Pentaerythritol tetrastearate | 0.0 | 0.0 | 0.0 | 0.0 | 0.0 |
| | | Stearic acid | 0.0 | 0.0 | 0.0 | 0.0 | 0.0 |
| | Precursor | Precursor A160S | 1.4 | 1.4 | 1.4 | 1.4 | 1.4 |
| | Coupler | Coupler A79 | 0.0 | 0.0 | 0.0 | 0.0 | 0.0 |
| | | Coupler A94 | 0.9 | 0.9 | 0.9 | 0.9 | 0.9 |
| | balancing agent | Cornstarch | 26.5 | 22.9 | 18.8 | 26.5 | 26.5 |
| | Fluidity enhancer | Silica | 3.0 | 3.0 | 3.0 | 3.0 | 3.0 |
| | Chelating reagent | EDTA | 2.2 | 2.2 | 2.2 | 2.2 | 2.2 |
| | Oil | Isopropyl myristate | 5.0 | 5.0 | 5.0 | 5.0 | 5.0 |
| | Total amount | | 100.0 | 45.0 | 45.0 | 100.0 | 100.0 |
| Coating material/solid alkaline agent | | | 0.11 | 0.18 | 0.25 | 0.11 | 0.11 |
| Content of coating material (% by mass) | | | 6.1 | 9.7 | 13.8 | 6.1 | 6.1 |
| Average particle size of coated solid alkaline agent (μm) | | | 100.6 | 180.2 | 384.2 | 82.4 | 88.9 |
| In the table, numerical values of the components are based on parts by mass, and "PC" means "Powder Composition for Hair Dye". | | | | | | | |

Table 1-1 (continued)

| | Production Examples | | 6 | 7 | 8 | 9 |
|---|---|---|---|---|---|---|
| PC | Solid alkaline agent | Trisodium phosphate (70.7 μm) | 55.0 | 55.0 | 55.0 | 55.0 |
| | | Trisodium phosphate (170.3 μm) | 0.0 | 0.0 | 0.0 | 0.0 |
| | | Sodium metasilicate (86.0 μm) | 0.0 | 0.0 | 0.0 | 0.0 |
| | Coating material | Stearyl stearate | 0.0 | 0.0 | 0.0 | 0.0 |
| | | Behenyl behenate | 0.0 | 0.0 | 0.0 | 0.0 |
| | | Stearyl alcohol | 0.0 | 0.0 | 0.0 | 0.0 |
| | | Behenyl alcohol | 6.1 | 0.0 | 0.0 | 0.0 |
| | | Paraffine Wax-140 | 0.0 | 6.1 | 0.0 | 0.0 |
| | | Paraffine Wax 9ND | 0.0 | 0.0 | 6.1 | 0.0 |
| | | Pentaerythritol tetrastearate | 0.0 | 0.0 | 0.0 | 6.1 |
| | | Stearic acid | 0.0 | 0.0 | 0.0 | 0.0 |
| | Precursor | Precursor A160S | 1.4 | 1.4 | 1.4 | 1.4 |
| | Coupler | Coupler A79 | 0.0 | 0.0 | 0.0 | 0.0 |
| | | Coupler A94 | 0.9 | 0.9 | 0.9 | 0.9 |
| | balancing agent | Cornstarch | 26.5 | 26.5 | 26.5 | 26.5 |
| | Fluidity enhancer | Silica | 3.0 | 3.0 | 3.0 | 3.0 |
| | Chelating reagent | EDTA | 2.2 | 2.2 | 2.2 | 2.2 |
| | Oil | Isopropyl myristate | 5.0 | 5.0 | 5.0 | 5.0 |
| | Total amount | | 100.0 | 100.0 | 100.0 | 100.0 |
| Coating material/solid alkaline agent | | | 0.11 | 0.11 | 0.11 | 0.11 |
| Content of coating material (% by mass) | | | 6.1 | 6.1 | 6.1 | 6.1 |
| Average particle size of coated solid alkaline agent (μm) | | | 100.1 | 77.9 | 98.7 | 99.9 |
| In the table, numerical values of the components are based on parts by mass, and "PC" means "Powder Composition for Hair Dye". | | | | | | |

Table 1-2

| | Production Examples | | 10 | 11 | 12 | 13 |
|---|---|---|---|---|---|---|
| PC | Solid alkaline agent | Trisodium phosphate (70.7 μm) | 55.0 | 55.0 | 0.0 | 0.0 |
| | | Trisodium phosphate (170.3 μm) | 0.0 | 0.0 | 55.0 | 55.0 |
| | | Sodium metasilicate (86.0 μm) | 0.0 | 0.0 | 0.0 | 0.0 |
| | Coating material | Stearyl stearate | 0.0 | 0.0 | 0.0 | 0.0 |
| | | Behenyl behenate | 0.0 | 0.0 | 0.0 | 0.0 |
| | | Stearyl alcohol | 0.0 | 0.0 | 6.1 | 0.0 |
| | | Behenyl alcohol | 0.0 | 0.0 | 0.0 | 0.0 |
| | | Paraffine Wax-140 | 0.0 | 0.0 | 0.0 | 0.0 |
| | | Paraffine Wax 9ND | 0.0 | 0.0 | 0.0 | 0.0 |
| | | Pentaerythritol tetrastearate | 0.0 | 0.0 | 0.0 | 0.0 |
| | | Stearic acid | 0.0 | 6.1 | 0.0 | 0.0 |
| | Surface modifier | Cornstarch | 0.0 | 0.0 | 0.0 | 0.0 |
| | Precursor | Precursor A160S | 1.4 | 1.4 | 0.7 | 0.7 |
| | Coupler | Coupler A79 | 0.0 | 0.0 | 0.0 | 0.0 |
| | | Coupler A94 | 0.9 | 0.9 | 0.4 | 0.4 |
| | balancing agent | Cornstarch | 32.6 | 26.5 | 27.6 | 33.7 |
| | Fluidity enhancer | Silica | 3.0 | 3.0 | 3.0 | 3.0 |
| | Chelating reagent | EDTA | 2.2 | 2.2 | 2.2 | 2.2 |
| | Oil | Isopropyl myristate | 5.0 | 5.0 | 5.0 | 5.0 |
| | Total amount | | 100.0 | 100.0 | 100.0 | 100.0 |
| Coating material/solid alkaline agent | | | 0.00 | 0.11 | 0.11 | 0.00 |
| Content of coating material (% by mass) | | | 0.0 | 6.1 | 6.1 | 0.0 |
| Average particle size of coated solid alkaline agent (μm) | | | 70.7 | 104.0 | 268.9 | 170.3 |
| In the table, numerical values of the components are based on parts by mass, and "PC" means "Powder Composition for Hair Dye". | | | | | | |

Table 1-2 (continued)

| | Production Examples | | 14 | 15 | 16 | 17 |
|---|---|---|---|---|---|---|
| PC | Solid alkaline agent | Trisodium phosphate (70.7 μm) | 55.0 | 55.0 | 0.0 | 0.0 |
| | | Trisodium phosphate (170.3 μm) | 0.0 | 0.0 | 0.0 | 55.0 |
| | | Sodium metasilicate (86.0 μm) | 0.0 | 0.0 | 24.4 | 0.0 |
| | Coating material | Stearyl stearate | 0.0 | 0.0 | 0.0 | 0.0 |
| | | Behenyl behenate | 0.0 | 0.0 | 0.0 | 0.0 |
| | | Stearyl alcohol | 0.0 | 0.0 | 2.7 | 6.1 |
| | | Behenyl alcohol | 0.0 | 0.0 | 0.0 | 0.0 |
| | | Paraffine Wax-140 | 6.1 | 0.0 | 0.0 | 0.0 |
| | | Paraffine Wax 9ND | 0.0 | 0.0 | 0.0 | 0.0 |
| | | Pentaerythritol tetrastearate | 0.0 | 0.0 | 0.0 | 0.0 |
| | | Stearic acid | 0.0 | 0.0 | 0.0 | 0.0 |
| | Surface modifier | Cornstarch | 0.0 | 0.0 | 0.0 | 12.2 |
| | Precursor | Precursor A160S | 0.5 | 0.5 | 0.7 | 0.7 |
| | Coupler | Coupler A79 | 0.8 | 0.8 | 0.0 | 0.0 |
| | | Coupler A94 | 0.0 | 0.0 | 0.4 | 0.4 |
| | balancing agent | Cornstarch | 27.6 | 33.6 | 58.1 | 15.3 |
| | Fluidity enhancer | Silica | 3.0 | 3.0 | 3.0 | 3.0 |
| | Chelating reagent | EDTA | 2.2 | 2.2 | 2.2 | 2.2 |
| | Oil | Isopropyl myristate | 5.0 | 5.0 | 5.0 | 5.0 |
| | Total amount | | 100.0 | 100.0 | 100.0 | 100.0 |
| Coating material/solid alkaline agent | | | 0.11 | 0.00 | 0.11 | 0.11 |
| Content of coating material (% by mass) | | | 6.1 | 6.1 | 2.7 | 6.1 |
| Average particle size of coated solid alkaline agent (μm) | | | 77.9 | 70.7 | 157.0 | 193.4 |
| In the table, numerical values of the components are based on parts by mass, and "PC" means "Powder Composition for Hair Dye". | | | | | | |

From the results of Production Examples 12 and 17, it is understood that an increase in the particle size of the solid composition can be inhibited by using a surface modifier that does not act as a binder. A reason thereof is considered that binding of the solid alkaline agents with each other by the coating material was able to be inhibited by using the surface modifier. Therefore, by combining the coating material and the surface modifier, coating of the solid alkaline agent and inhibition of an excess granulation during the production are possible.

[Evaluation Method of Solid Composition]

The solid composition produced as the above was stored at 40°C and 25%RH. Humidification under a condition of 25°C, 60%RH, and 16 hours was performed every one week after the start of the storage. The following evaluations were performed when two weeks or four weeks had elapsed after the start of the storage. The humidification was performed once before the evaluation after two weeks, and three times before the evaluation after four weeks.

The evaluations of the stored solid composition were performed by subjecting tress (goat hair: LB-W) to the following operations.

As the liquid composition containing the oxidizing agent, a liquid composition having the proportioning indicated in Table 2 was used. Precisely 0.5 g of the solid composition and 4.5 g of the liquid composition were weighed and taken (so that the mass rate satisfied solid composition: liquid composition = 1:9) in a screw tube, followed by mixing with a

vortex mixer for about 10 seconds to prepare paste. Tress was put on a tray, and 1.5 g of the obtained paste was dropped and carefully applied using a spatula so that the paste soaked into the whole hair bundle. The tress after the application was covered with Saran Wrap (trademark) and left still for 30 minutes in an electric drier at 40°C. Then, the tress was taken out of the electric drier and the both sides of the tress were rinsed for 10 strokes each with warm water at 40°C. The tress after rinsing was washed with shampoo for 15 seconds and the both sides of the tress were rinsed again for 10 strokes each with warm water at 40°C. For the evaluation of dyeability, a spectral colorimeter CM-700d manufactured by Konica Minolta, Inc. was used. For one bundle of tress, six points in total including three points on the front side and three points on the back side were measured in color, and an average value of values represented by L*a*b* color system was calculated.

Storage stability of the solid composition was evaluated using a color difference ΔE between dyeability using the solid composition immediately after the production and dyeability using the solid composition two weeks or four weeks after the start of storage. The color difference ΔE can be calculated by the following formula (1).

$$\Delta E = \sqrt{(\Delta L)^2 + (\Delta a)^2 + (\Delta b)^2} \qquad \text{Formula (1)}$$

In the formula (1), each of ΔL, Δa, and Δb is a difference of average values calculated from the measured values of dyeability using the solid composition immediately after the production and dyeability using the solid composition two weeks or four weeks after the start of storage.

Effect of the coating material of the solid composition on dyeability was evaluated using a color difference ΔE0 between dyeabilities using the solid composition of Examples immediately after the production and the solid composition of Comparative Examples immediately after the production. The color difference ΔE0 can be calculated by the above formula (1), and in the formula (1), each of ΔL, Δa, and Δb is a difference of average values calculated from the measured values of dyeability using the solid composition of Examples immediately after the production and dyeability using the solid composition of Comparative Examples immediately after the production. The smaller ΔE0 is, the smaller the effect of the coating material on dyeability is.

Table 2

| Liquid composition containing oxidizing agent | % by mass |
|---|---|
| 85% Phosphoric acid | 0.19 |
| Monoethanolamine | 2.78 |
| 47% Sulfuric acid | 4.50 |
| 30% Hydrogen peroxide water | 11.11 |
| 10% Sulfuric acid | 0.91 |
| Water | 80.51 |
| Total | 100.00 |

Dyeability was measured in accordance with the above-described method, using the solid compositions produced in Production Examples. Evaluation bases depend on the coupler used, and the results are indicated in Table 3.

Examples 1 to 10, 12, and 13, and Comparative Examples 1 to 3

Evaluation basis for Examples 1 to 10, 12, and 13, and Comparative Examples 1 to 3, in which A94 (2-chloro-3-amino-6-methylphenol) was used as the coupler and trisodium phosphate was used as the solid alkaline agent, is as below and in the case where the evaluation result is equal to or better than C, the effect of the present invention is exhibited.

A: ΔE after two-week storage is 6 or less and ΔE after four-week storage is 10 or less.
B: ΔE after two-week storage is 6 or less and ΔE after four-week storage is more than 10 and 16 or less.
C: ΔE after two-week storage is more than 6 and 8 or less and ΔE after four-week storage is 20 or less.
D: ΔE after two-week storage is more than 8 or ΔE after four-week storage is more than 20.

Example 11 and Comparative Example 4

Evaluation basis for Example 11 and Comparative Example 4, in which A79 (1,3-bis(2,4-diaminophenoxy)propane) was used as the coupler and trisodium phosphate was used as the solid alkaline agent, is as below and in the case where the evaluation result is equal to or better than C, the effect of the present invention is exhibited.

A: ΔE's after two-week storage and after four-week storage are 5 or less.
B: ΔE after two-week storage is 5 or less and ΔE after four-week storage is more than 5 and 7 or less.
C: ΔE after two-week storage is 5 or less and ΔE after four-week storage is more than 7 and 9 or less.
D: ΔE after two-week storage is more than 5.

Table 3

| | | Solid alkaline agent | Particle size of solid alkaline agent (μm) | Coupler | Coating material | Melting point (°C) |
|---|---|---|---|---|---|---|
| Example 1 | PE 1 | Trisodium phosphate | 70.7 | A94 | Stearyl stearate | 60 |
| Example 2 | PE 2 | | 70.7 | | Stearyl stearate | 60 |
| Example 3 | PE 3 | | 70.7 | | Stearyl stearate | 60 |
| Example 4 | PE 4 | | 70.7 | | Behenyl behenate | 70 |
| Example 5 | PE 5 | | 70.7 | | Stearyl alcohol | 59 |
| Example 6 | PE 6 | | 70.7 | | Behenyl alcohol | 69 |
| Example 7 | PE 7 | | 70.7 | | Paraffine Wax-140 | 61 |
| Example 8 | PE 8 | | 70.7 | | Paraffine Wax 9ND | 75 |
| Example 9 | PE 9 | | 70.7 | | Pentaerythritol tetrastearate | 62 |
| Comparative Example 1 | PE 10 | | 70.7 | | None | - |
| Comparative Example 2 | PE 11 | | 70.7 | | Stearic acid | 69 |
| Example 10 | PE 12 | | 170.3 | | Stearyl alcohol | 59 |
| Comparative Example 3 | PE 13 | | 170.3 | | None | - |
| Example 11 | PE 14 | | 70.7 | A79 | Paraffine Wax-140 | 61 |
| Comparative Example 4 | PE 15 | | 70.7 | | None | - |
| Example 12 | PE 16 | Sodium metasilicate | 86 | A94 | Stearyl alcohol | 59 |

(continued)

| | | Solid alkaline agent | Particle size of solid alkaline agent (μm) | Coupler | Coating material | Melting point (°C) |
|---|---|---|---|---|---|---|
| Example 13 | PE 17 | Trisodium phosphate | 170.3 | A94 | Stearyl alcohol + surface modifier (cornstarch) | 59 |

In the table, "PE" means "Production Example".
*1: ΔE0 is a value of the difference between dyeabilities immediately after the production of Examples 1 to 9 and Comparative Example 1 obtained by using formula (1).
*2: A value of the difference between dyeabilities immediately after the production of Examples 10 and 13, and Comparative Example 3 obtained by using formula (1).
*3: A value of the difference between dyeabilities immediately after the production of Example 11 and Comparative Example 4 obtained by using formula (1).
*4: A mass rate of the surface modifier relative to the solid alkaline agent (surface modifier/solid alkaline agent) is 0.22.

Table 3 continued

| | Coating material/solid alkaline agent | Immediately after production | | | After 2 weeks | | | After 4 weeks | | |
|---|---|---|---|---|---|---|---|---|---|---|
| | | L value | a value | b value | L value | a value | b value | L value | a value | b value |
| Example 1 | 0.11 | 41.2 | 16.8 | -14.8 | 41.3 | 16.0 | -14.2 | 50.4 | 13.5 | -10.9 |
| Example 2 | 0.18 | 38.0 | 17.4 | -15.0 | 39.2 | 16.7 | -14.5 | 45.2 | 15.2 | -13.1 |
| Example 3 | 0.25 | 38.6 | 17.3 | -15.3 | 40.2 | 16.4 | -14.3 | 45.3 | 15.1 | -12.8 |
| Example 4 | 0.11 | 38.0 | 17.4 | -15.2 | 41.6 | 16.6 | -14.1 | 44.7 | 14.8 | -11.4 |
| Example 5 | 0.11 | 41.4 | 16.5 | -14.7 | 42.9 | 15.6 | -13.5 | 50.9 | 12.2 | -8.8 |
| Example 6 | 0.11 | 38.0 | 17.5 | -15.4 | 43.2 | 16.4 | -14.0 | 46.7 | 14.3 | -11.0 |
| Example 7 | 0.11 | 38.7 | 17.0 | -14.3 | 42.9 | 15.0 | -11.9 | 51.1 | 12.2 | -8.4 |
| Example 8 | 0.11 | 40.2 | 17.0 | -14.4 | 43.2 | 15.1 | -12.2 | 48.0 | 13.0 | -9.2 |
| Example 9 | 0.11 | 39.3 | 17.5 | -15.3 | 42.4 | 15.9 | -13.3 | 45.4 | 14.3 | -10.6 |
| Comparative Example 1 | 0.00 | 38.7 | 16.7 | -15.4 | 50.1 | 12.9 | -9.6 | 57.3 | 9.3 | -3.7 |
| Comparative Example 2 | 0.11 | 42.0 | 16.7 | -15.2 | 48.8 | 13.0 | -9.8 | 55.1 | 9.9 | -5.1 |
| Example 10 | 0.11 | 47.3 | 15.3 | -12.7 | 54.2 | 13.1 | -10.6 | 61.9 | 9.3 | -5.0 |
| Comparative Example 3 | 0.00 | 47.9 | 15.4 | -13.4 | 62.6 | 8.5 | -3.6 | 66.5 | 7.0 | -1.2 |
| Example 11 | 0.11 | 50.3 | 3.0 | -12.7 | 53.6 | 2.3 | -11.4 | 52.9 | 2.2 | -11.4 |
| Comparative Example 4 | 0.00 | 51.0 | 2.6 | -12.0 | 56.4 | 1.9 | -10.4 | 59.5 | 1.5 | -8.6 |
| Example 12 | 0.11 | 49.8 | 14.7 | -13.6 | 48.6 | 15.0 | -12.7 | 51.6 | 14.0 | -11.7 |

(continued)

| | Coating material/solid alkaline agent | Immediately after production | | | After 2 weeks | | | After 4 weeks | | |
|---|---|---|---|---|---|---|---|---|---|---|
| | | L value | a value | b value | L value | a value | b value | L value | a value | b value |
| Example 13 | 0.11*4 | 49.0 | 15.4 | -14.0 | 51.3 | 12.4 | -10.5 | 57.5 | 11.5 | -8.0 |

*1: ΔE0 is a value of the difference between dyeabilities immediately after the production of Examples 1 to 9 and Comparative Example 1 obtained by using formula (1).
*2: A value of the difference between dyeabilities immediately after the production of Examples 10 and 13, and Comparative Example 3 obtained by using formula (1).
*3: A value of the difference between dyeabilities immediately after the production of Example 11 and Comparative Example 4 obtained by using formula (1).
*4: A mass rate of the surface modifier relative to the solid alkaline agent (surface modifier/solid alkaline agent) is 0.22.

Table 3 (continued)

| | ΔE0*1 between Example and Comparative Example immediately after production | ΔE between immediately after production and after 2 weeks | ΔE between immediately after production and after 4 weeks | Evaluation |
|---|---|---|---|---|
| Example 1 | 2.5 | 1.0 | 10.6 | B |
| Example 2 | 1.1 | 1.5 | 7.8 | A |
| Example 3 | 0.6 | 2.1 | 7.4 | A |
| Example 4 | 1.1 | 3.8 | 8.1 | A |
| Example 5 | 2.7 | 2.1 | 12.0 | B |
| Example 6 | 1.1 | 5.5 | 10.3 | B |
| Example 7 | 1.1 | 5.3 | 15.9 | B |
| Example 8 | 1.8 | 4.2 | 12.6 | B |
| Example 9 | 1.0 | 4.0 | 8.4 | A |
| Comparative Example 1 | - | 13.3 | 23.2 | D |
| Comparative Example 2 | 3.3 | 9.5 | 17.9 | D |
| Example 10 | 0.94*2 | 7.6 | 17.8 | C |
| Comparative Example 3 | - | 19.3 | 24.2 | D |
| Example 11 | 1.08*3 | 3.6 | 3.0 | A |
| Comparative Example 4 | - | 5.7 | 9.3 | D |
| Example 12 | - | 1.5 | 4.6 | A |

(continued)

| | ΔE0*1 between Example and Comparative Example immediately after production | ΔE between immediately after production and after 2 weeks | ΔE between immediately after production and after 4 weeks | Evaluation |
|---|---|---|---|---|
| Example 13 | 1.2*2 | 5.4 | 11.6 | B |
| *1: ΔE0 is a value of the difference between dyeabilities immediately after the production of Examples 1 to 9 and Comparative Example 1 obtained by using formula (1).<br>*2: A value of the difference between dyeabilities immediately after the production of Examples 10 and 13, and Comparative Example 3 obtained by using formula (1).<br>*3: A value of the difference between dyeabilities immediately after the production of Example 11 and Comparative Example 4 obtained by using formula (1).<br>*4: A mass rate of the surface modifier relative to the solid alkaline agent (surface modifier/solid alkaline agent) is 0.22. | | | | |

From the evaluation results of Examples and Comparative Examples, it is understood that storage stability of the solid composition is improved and a decrease in dyeability is inhibited by coating the surface of the solid alkaline agent with the coating material.

From the results of Examples 1 to 3, it is understood that storage stability of the solid composition is further improved by increasing the content of the coating material. According to the results of Examples 1, 4, and 9, when an ester is used as the coating material and the melting point of the ester is high, storage stability of the solid composition is improved and a decrease in dyeability is inhibited.

From the results of Examples 5 and 10, it is understood that the smaller the particle size of the solid alkaline agent is, the more storage stability of the solid composition is improved. When the average particle size of the solid alkaline agent is large, a surface area of the solid alkaline agent as the whole decreases, and thus the required amount of the coating material decreases. Therefore, in view of the results of Examples 1 to 3, it had been expected that the larger the average particle size of the solid alkaline agent was, the more storage stability of the solid composition would be improved. However, the result was contrary to the expectations. A reason thereof is considered that when the average particle size of the solid alkaline agent is large, a surface area per one particle increases, and the solid alkaline agent is not uniformly coated with the coating material.

From the results of Example 5 and Example 12, it is understood that both trisodium phosphate and sodium metasilicate can be preferably used as the solid alkaline agent. It is expected that also when an ester such as stearyl stearate is used as the coating material to coat sodium metasilicate, storage stability is similarly improved.

There was almost no difference between dyeabilities immediately after the production of Examples 1 to 9 and Comparative Example 1, Example 10 and Comparative Example 3, and Example 11 and Comparative Example 4. Therefore, unexpectedly, it was understood that coating the solid alkaline agent had almost no effect on dyeability.

It is considered that in Comparative Example 2, the fatty acid formed a salt (soap) with trisodium phosphate, the salt of the fatty acid coating the surface of the solid alkaline agent was dissolved with the moisture in the air during storage, and therefore storage stability of the solid composition was low.

From the results of Examples 10 and 13, it is understood that storage stability is improved by further using cornstarch as the surface modifier. A reason thereof is considered that, by attaching powder cornstarch on the surface of the solid alkaline agent coated with stearyl alcohol, an inhibition effect on a contact between the solid alkaline agent and the oxidation dye precursor was improved.

Industrial Applicability

According to the present invention, a solid composition excellent in storage stability and a method for producing the same, a hair dye kit including the solid composition, use of the solid composition for a cosmetic, and a method for inhibiting a reaction between a dye and a solid alkaline agent can be provided. The solid composition of the present invention can be used for makeup cosmetics, and hair treatment agents such as hair dyes, permanent wave agents, and treatments.

## Claims

**1.** A solid composition for dyeing keratin, comprising a dye and a coated solid alkaline agent, wherein at least a part

of a surface of the solid alkaline agent is coated with a coating material having a melting point of 30°C or more and containing one or more selected from an alcohol, an ether, an ester, and a hydrocarbon.

**2.** The solid composition for dyeing according to claim 1, wherein a mass rate of the coating material relative to a mass of the solid alkaline agent (coating material/solid alkaline agent) is 0.01 or more and 1 or less.

**3.** The solid composition for dyeing according to claim 1 or 2, wherein the solid alkaline agent contains one or more selected from an orthosilicate, a pyrosilicate, a metasilicate, a phosphate, a metadisilicate, a carbonate, and a hydroxide.

**4.** The solid composition for dyeing according to any one of claims 1 to 3, wherein a solubility of the coating material to water at 25°C is 100 mg/100 g or less.

**5.** The solid composition for dyeing according to any one of claims 1 to 4, wherein a compound used for the coating material has 16 or more carbon atoms.

**6.** The solid composition for dyeing according to any one of claims 1 to 5, wherein the compound used for the coating material has a monovalent hydrocarbon group having 14 or more carbon atoms.

**7.** The solid composition for dyeing according to any one of claims 1 to 6, wherein the dye contains one or more selected from a precursor, a coupler, and a direct dye.

**8.** The solid composition for dyeing according to claim 7, wherein the precursor contains one or more selected from a phenylenediamine compound, an aminophenol compound, and a salt thereof.

**9.** The solid composition for dyeing according to claim 7 or 8, wherein the precursor contains one or more selected from 2-methoxymethylparaphenylenediamine, N-methoxyethyl-paraphenylenediamine, paraaminophenol, and a salt thereof.

**10.** The solid composition for dyeing according to any one of claims 7 to 9, wherein the coupler contains one or more selected from an aromatic amine compound, a phenol compound, a pyridine compound, and a salt thereof.

**11.** The solid composition for dyeing according to any one of claims 7 to 9, wherein the coupler contains one or more selected from 1,3-bis(2,4-diaminophenoxy)propane, 2-chloro-3-amino-6-methylphenol, 4-chlororesorcinol, 2-methylresorcinol, 2-amino-3-hydroxypyridine, 2,4-diaminophenoxyethanol, 2-methyl-5-amino-6-chlorophenol, 2-amino-4-hydroxyethylaminoanisole, 5-amino-6-chloro-o-cresol, 3-amino-2,4-dichlorophenol, and a salt thereof.

**12.** The solid composition for dyeing according to any one of claims 1 to 11, wherein an average particle size of the solid alkaline agent is 10 μm or more and 5 mm or less.

**13.** The solid composition for dyeing according to any one of claims 1 to 12, wherein a content of water in the solid composition is 10% by mass or less.

**14.** The solid composition for dyeing according to any one of claims 1 to 13, which is a powder composition.

**15.** The solid composition for dyeing according to any one of claims 1 to 14, stored in ajar-type container.

**16.** A hair dye kit comprising the solid composition for dyeing according to any one of claims 1 to 15 and a liquid composition containing an oxidizing agent.

**17.** The hair dye kit according to claim 16, wherein the oxidizing agent contains hydrogen peroxide water.

**18.** A method for producing the solid composition according to any one of claims 1 to 14, comprising the following Steps 1 and 2:

Step 1: a step of mixing the coating material that has been melted and the solid alkaline agent to obtain a coated material of the solid alkaline agent; and
Step 2: a step of mixing the coated material obtained and a dye.

**19.** Use of a solid composition for dyeing for a cosmetic, the solid composition comprising a dye and a coated solid alkaline agent, wherein at least a part of a surface of the solid alkaline agent is coated with a coating material having a melting point of 30°C or more and containing one or more selected from an alcohol, an ether, an ester, and a hydrocarbon.

**20.** The use according to claim 19, wherein the cosmetic is a hair treatment agent.

**21.** A method for inhibiting a reaction between a dye and a coated solid alkaline agent, comprising using the coated solid alkaline agent in which at least a part of a surface of the solid alkaline agent is coated with a coating material containing one or more selected from an alcohol, an ether, an ester, and a hydrocarbon.

**INTERNATIONAL SEARCH REPORT**

International application No. **PCT/JP2022/035067**

**A. CLASSIFICATION OF SUBJECT MATTER**

***A61K 8/25***(2006.01)i; ***A61K 8/24***(2006.01)i; ***A61K 8/31***(2006.01)i; ***A61K 8/33***(2006.01)i; ***A61K 8/34***(2006.01)i; ***A61K 8/37***(2006.01)i; ***A61K 8/41***(2006.01)i; ***A61Q 5/10***(2006.01)i

FI: A61K8/25; A61K8/24; A61K8/31; A61K8/33; A61K8/34; A61K8/37; A61K8/41; A61Q5/10

According to International Patent Classification (IPC) or to both national classification and IPC

**B. FIELDS SEARCHED**

Minimum documentation searched (classification system followed by classification symbols)

A61K8/25; A61K8/24; A61K8/31; A61K8/33; A61K8/34; A61K8/37; A61K8/41; A61Q5/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | EP 1752191 A1 (HENKEL KOMMANDITGESELLSCHAFT AUF AKTIEN) 14 February 2007 (2007-02-14)<br>claims, paragraphs [0022], [0023], [0025], [0029], [0031], [0039] | 1-21 |
| X | JP 2019-055941 A (KAO CORP.) 11 April 2019 (2019-04-11)<br>claims, paragraphs [0004], [0032]-[0036], [0117] | 1-2, 5, 7-21 |
| X | DE 102004038991 A1 (HENKEL KGAA) 23 February 2006 (2006-02-23)<br>claims, paragraphs [0032]-[0036], [0040] | 1-5, 7-21 |
| A | US 2012/0009134 A1 (WELZ, Carolin et al.) 12 January 2012 (2012-01-12)<br>entire text | 1-21 |
| A | WO 2021/083903 A1 (L' OREAL) 06 May 2021 (2021-05-06)<br>entire text | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 November 2022** | **29 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/035067**

| Patent document cited in search report | Publication date (day/month/year) | Patent family member(s) | Publication date (day/month/year) |
|---|---|---|---|
| EP 1752191 A1 | 14 February 2007 | DE 102005038073 A1 | |
| | | AT 407723 T | |
| JP 2019-055941 A | 11 April 2019 | US 2020/0214942 A1<br>claims, paragraphs [0004], [0038]-[0042], [0082] | |
| | | WO 2019/059252 A1 | |
| | | EP 3685816 A1 | |
| | | CN 111093596 A | |
| | | RU 2020113705 A | |
| DE 102004038991 A1 | 23 February 2006 | WO 2006/018099 A1 | |
| | | EP 1776085 A1 | |
| | | AT 465711 T | |
| US 2012/0009134 A1 | 12 January 2012 | WO 2010/105906 A2 | |
| | | EP 2563317 A2 | |
| | | DE 102009001636 A1 | |
| WO 2021/083903 A1 | 06 May 2021 | FR 3102361 A1 | |
| | | KR 10-2022-0070274 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2019151615 A
- EP 1752191 A

### Non-patent literature cited in the description

- *Journal of The Chemical Society of Japan,* 1982, (11), 2116-2119, 1830-1834